Europäisches Patentamt

European Patent Office

Office européen des brevets

(11) Veröffentlichungsnummer: **0 228 568 B1**

# EUROPÄISCHE PATENTSCHRIFT

(45) Veröffentlichungstag der Patentschrift: 16.10.91

(21) Anmeldenummer: 86116314.5

(22) Anmeldetag: 25.11.86

(51) Int. Cl.5: **A61K 39/44**, C12N 11/00, C12N 11/02, A61B 19/00

(54) Trägerkörper, der durch kovalent an seine Oberfläche gebundene Antikörper bioaktiviert ist.

(30) Priorität: 05.12.85 DE 3543066
15.04.86 DE 3612643

(43) Veröffentlichungstag der Anmeldung:
15.07.87 Patentblatt 87/29

(45) Bekanntmachung des Hinweises auf die
Patenterteilung:
16.10.91 Patentblatt 91/42

(84) Benannte Vertragsstaaten:
CH DE FR GB IT LI

(56) Entgegenhaltungen:
EP-A- 0 089 880        EP-A- 0 122 833
DE-A- 2 938 636        FR-A- 2 554 340
FR-A- 2 564 732        SE-A- 8 402 662

NATURE, Mai 1984, vol. 309, S. 385

J.DENT.RES., 1971, Heft 50, S. 1392-1406

SCIENCE, vol. 242, 16.12.88, S. 1528-1533

(73) Patentinhaber: Anawa München Aktiengesellschaft Biologische Laboratorien
Behringstrasse 6
W-8033 Planegg(DE)

(72) Erfinder: Müller-Lierheim, Wolfgang G.K., Dr.
Lichtweg 5
W-8032 Gräfelfing(DE)

(74) Vertreter: Nöth, Heinz, Dipl.-Phys. et al
Patentanwälte Pfenning, Meinig & Partner
Mozartstrasse 17
W-8000 München 2(DE)

## Beschreibung

Die Erfindung betrifft einen Implantatkörper, der durch kovalent an seine Oberfläche gebundene Antikörper bioaktiviert ist.

Es ist bekannt, biologisch aktive Verbindungen, wie beispielsweise Proteine, Enzyme, Antikörper, an modifizierte Trägermaterialien zu immobilisieren (DDR-PS 210 693), und in der Medizin, Pharmazie, Biotechnologie, Chemie, Landwirtschaft usw. einzusetzen. Ein breites Anwendungsfeld finden derartig beispielsweise durch kovalente Bindung an Trägerkörper immobilisierte Antikörper bei der Bestimmung von Nachweisreaktionen, beispielsweise zur Bestimmung von in Körperflüssigkeiten vorhandenen Substanzen wie Harmonen (DE-AS 22 06 103). Ferner ist aus der FR-PS 2 554 349 bekannt, mittels spezifischer monoklonaler, an einem Trägerkörper kovalent gebundener Antikörper ein entsprechendes Antikoagulans, beispielsweise Heparin, zu binden. Derart ausgebildete Trägerkörper können als Prothesen verwendet werden.

Ferner wird in der älteren, als DE-OS 35 19 073 veröffentlichten deutschen Patentanmeldung ein künstliches knochenerzeugendes Biomaterial vorgeschlagen, das ein Gemisch aus einem knochenerzeugenden Faktor und einen ersten Träger für den Faktor enthält, wobei der Träger ausgewählt wird unter Collagen, seinen Derivaten und denaturierten Substanzen.

Aufgabe der Erfindung ist es im Gegensatz dazu, die Oberflächenbeschaffenheit des eingangs genannten Implantatkörper so zu gestalten, daß darauf eine Immobilisierung der Knorpel-und Knochenbildende Proteine erzeilt wird.

Diese Aufgabe wird beim eingangs genannten Implantatkörper erfindungsgemäß dadurch gelöst, daß ein knochenimplantatkörper hergestellt wird, der eine aus einem Polymerisat bestehende Oberfläche aufweist an der durch Kovalente Bindung monoklonale Antikörper bzw. Antikörper, fragmente angelagert sind, die für im menschlichen Körper natürlicherweise vorkommende Knorper-und Knochenbildende Proteine Rezeptoren bilden.

Hochspezifische monoklonale Antikörper können mit den derzeit verfügbaren Techniken gegen nahezu jedes gewünschte Epitop (determinante Gruppe) entwickelt werden. Für die Entwicklung monoklonaler Antikörper genügen geringe Antigenmengen. Im Gegensatz zu einer Vielzahl körpereigener Substanzen lassen sich monoklonale Antikörper in Kilogramm-Mengen wirtschaftlich produzieren, ohne daß es dazu der gentechnologischen Modifikation von Bakterien oder Säugerzellen bedarf. Monoklonale Antikörper oder deren Fragmente lassen sich reproduzierbar kovalent an Festkörperoberflächen binden. Nach proteolytischer Spaltung der Antikörper, beispielsweise mittels Papain, und Reinigung, beispielsweise durch Elektrophorese oder Chromatographie, lassen sich die Peptidketten, insbesondere die variablen Teile der Peptidketten, welche die Bindungsorte an ihren N-terminalen Enden aufweisen, kovalent an die Implantatoberfläche binden. Die kovalente Bindung kann über in der Implantatoberfläche vorhandene Oxirangruppen erfolgen. Die Implantatoberfläche wird hierzu von einem geeigneten Polymerisat gebildet. Andere geeignete kovalente Bindungsarten sind beispielsweise aus Chemiker-Zeitung, 97. Jahrgang (1973), Nr. 11, Seite 612 bekannt.

Durch die erfindungsgemäße Ausgestaltung der Enden bzw. Bindungsorte der Peptidketten der Antikörper bzw. Antikörper bzw. Antikörperfragmente bilden diese Rezeptoren für im Körper vorkommende Knorpel-und Knochenbildende Proteine. Derartig an die Implantatoberfläche gebundene monoklonale Antikörper bzw. Antikörperfragmente sind stabil und werden auch über lange Zeiträume im menschlichen Körper nicht enzymatisch abgebaut. Die Spezifität ermöglicht es, körpereigene Moleküle an einem Epitop in definierter Weise so an die Implantatoberfläche zu adsorbieren, daß das aktive Zentrum des Moleküls optimal präsentiert wird. Durch adsorptive Bindung körpereigener Substanz an Implantatoberflächen, die mit monoklonalen Antikörpern beschichtet sind, kann erreicht werden, daß die Implantatoberflächen mit körpereigener Substanz "maskiert" werden.

Durch die Assoziationsfähigkeit der monoklonalen Antikörper gegenüber knochenbildenden Proteinen (Bone Morphogenetic Proteins) lassen sich stabile bioaktive Implantatoberflächen schaffen, bei denen nach Implantation die auf der Implantatoberfläche befindlichen Antikörper bzw. Antikörperfragmente das knochenbildende Protein des Patienten adsorptiv binden.

Zur Erläuterung der Erfindung wird im folgenden noch die Herstellung eines Ausführungsbeispiels, welches ein Implantat ist, beschrieben.

Ein Metallkern eines Implantatkörpers wird mit einem Copolymerisat überzogen, das zumindest in seiner Oberfläche Oxirangruppen aufweist, über die eine kovalente Bindung der Antikörper erreicht wird. Nachdem die Antikörper an die Oberfläche gebunden sind, wird noch Restmonomer aus der Copolymerisatoberfläche extrahiert. Vor der Bindungsreaktion werden die monoklonalen Antikörper gegebenenfalls durch Proteolyse gespalten und die variablen Regionen bzw. die abgespaltenen Peptidketten werden an der aktivierten Copolymerisatoberfläche kovalent gebunden. Diese kovalente Bindung erfolgt über Oxirangruppen.

Nach der Implantation wird das Implantat mit knochenbildenden Proteinen maskiert, wobei die knochenbildenden Proteine an die Antikörper an definierten Stellen (Epitopen) adsorbiert sind. Auf diese Weise läßt sich eine Immobilisierung der knochenbildenden Proteine an der Implantatoberfläche erzielen.

Die auf diese Weise hergestellte Implantatoberfläche besitzt eine hohe Stabilität gegenüber Spaltung durch Proteasen. Die knochenbildenden Proteine sind adsorptiv gebunden. Falls eine Spaltung der Proteine durch Proteasen erfolgt, werden diese insbesondere aufgrund der durch die Spaltung bedingten Konformationsänderungen durch intakte knochenbildende Proteine ersetzt. Die an die Antikörper adsorbierten Proteine behalten ihre volle Wirksamkeit bei, so daß sie ihre Wirksamkeit bei der Knochenbildung nach Einsetzen des Implantats voll entfalten können.

**Patentansprüche**

1. Implantat mit einem implantierbaren Implantatkörper, der eine aus einem Polymerisat bestehende Oberfläche aufweist, an der durch kovalente Bindung monoklonale Antikörper bzw. Antikörperfragmente angelagert sind, die für im menschlichen Körper natürlicherweise vorkommende Proteine Rezeptoren bilden, dadurch gekennzeichnet, daß der Implantatkörper ein Knochenimplantatkörper ist, und die Antikörper bzw. Antikörperfragmente Rezeptoren für nach der Implantation adsorptiv gebundene knorpel- und knochenbildende Proteine sind.

**Claims**

1. An implant comprising an implantable implant body having a surface which comprises a polymer and on which monoclonal antibodies or antibody fragments which form receptors for proteins naturally occurring in the human body are deposited by covalent bonding, characterised in that the implant body is a bone implant body and the antibodies or antibody fragments are receptors for cartilage- and bone-forming proteins which are adsorptively bonded after implantation.

**Revendications**

1. Implant avec un corps d'implant implantable qui présente une surface constituée d'un polymère et sur laquelle sont fixés par liaison covalente des anticorps et respectivement des fragments d'anticorps qui constituent des récepteurs pour des protéines naturellement présentes dans le corps humain, **caractérisé en ce** que le corps d'implant est un corps d'implant osseux et que les anticorps et respectivement les fragments d'anticorps constituent des récepteurs pour des protéines chondrogènes et ostéogènes liées de manière adsorbante après l'implantation.